# EUROPÄISCHE PATENTSCHRIFT

(11) **EP 0 948 482 B1**
(45) Veröffentlichungstag und Bekanntmachung des Hinweises auf die Patenterteilung: **03.04.2002**
(21) Anmeldenummer: 97952043.4
(22) Anmeldetag: 08.12.1997
(51) Int. Cl.: C07C 311/00

(54) **VERFAHREN ZUR HERSTELLUNG VON TRIFLUORACETESSIGSÄURE-ANILIDEN**
METHOD FOR PRODUCING TRIFLUOROACETOACETIC ACID ANILIDES
PROCEDE POUR LA PREPARATION D'ANILIDES D'ACIDE TRIFLUOROACETOACETIQUE

(30) Priorität: 19.12.1996 DE 19652955
(43) Veröffentlichungstag der Anmeldung: 13.10.1999
(73) Patentinhaber: BAYER AG, 51368 Leverkusen (DE)
(72) Erfinder: LANTZSCH, Reinhard, D-42115 Wuppertal (DE); STEINBECK, Karl, D-51399 Burscheid (DE); KÄMPFEN, Ulrich, CH-3900 Brig (CH)
(86) Internationale Anmeldenummer: EP9706842
(87) Internationale Veröffentlichungsnummer: WO9827057

(56) Entgegenhaltungen:
- EP-A- 0 648 772
- WO-A-95/32952
- DE-A- 19 543 676
- GB-A- 931 689
- US-A- 2 797 217
- T. KINOSHITA ET AL: CHEM. PHARM. BULL., Bd. 34, Nr. 4, 1986, Seiten 1809-1813, XP002063006
- T. KINOSHITA ET AL: CHEM. PHARM. BULL., Bd. 37, Nr. 8, 1989, Seiten 2026-2029, XP002063007

## Beschreibung

Die vorliegende Erfindung betrifft ein neues Verfahren zur Herstellung von Trifluoracetessigsäure-aniliden, die als Zwischenprodukte zur Herstellung von herbizid wirksamen Uracil-Derivaten verwendbar sind.

Es ist bereits bekannt geworden, daß sich Trifluoracetessigsäure-arylamide herstellen lassen, indem man Trifluoracetessigester mit Arylaminen umsetzt (vgl. DE-A 42 18 159, EP-A 0 598 436 und J. Het. Chem. 2 (1965), 124). Nachteilig an diesem Verfahren ist aber, daß es nicht breit anwendbar ist und die gewünschten Produkte nur in relativ niedrigen Ausbeuten anfallen, da störende Nebenreaktionen ablaufen. So greift das Arylamin in beträchtlichem Maße die Carbonylgruppe an, die der Trifluormethyl-Gruppe benachbart ist. Das dabei entstehende Enamin reagiert leicht mit einem weiteren Arylamin-Molekül, so daß Bis-Addukte erhalten werden. Diese Umsetzung kann durch das folgende Formelschema veranschaulicht werden. 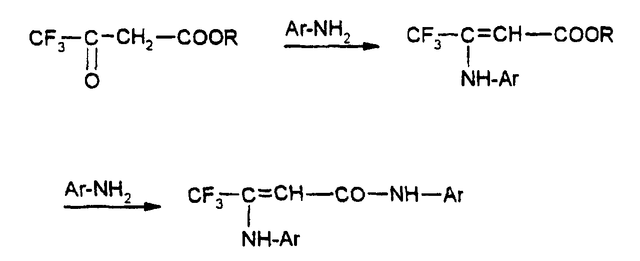 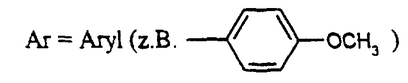 R = Alkyl (z.B. Methyl oder Ethyl)

Weiterhin ist bekannt, daß sich Trifluoracetessigsäure-arylamide herstellen lassen, indem man aus Bis-Addukten der zuvor angegebenen Struktur ein Molekül Arylamin abspaltet (vgl. DE-A 42 18 159). Ungünstig an diesem Verfahren ist, daß eine Stufe mehr erforderlich ist als bei der oben beschriebenen Methode. Außerdem sind die Ausbeuten auch hier für eine Durchführung in technischem Maßstab unbefriedigend.

Es wurde nun gefunden, daß man Trifluoracetessigsäure-anilide der Formel 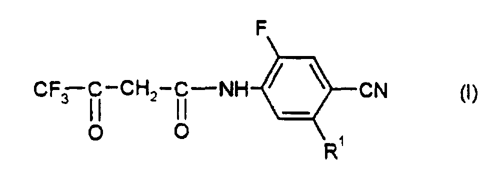 in welcher
- R¹: für Halogen oder einen Rest der Formel 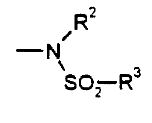 steht, worin
- R²: für Wasserstoff oder gegebenenfalls substituiertes Alkyl steht und
- R³: für gegebenenfalls substituiertes Alkyl, gegebenenfalls substituiertes Cycloalkyl oder für gegebenenfalls substituiertes Aryl steht,
erhält, wenn man Trifluoracetessigsäure-chlorid der Formel 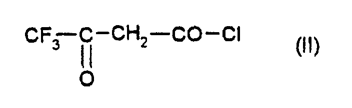 mit Anilinen der Formel 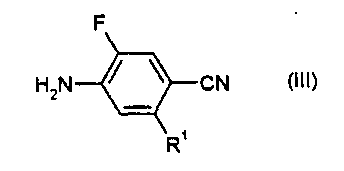 in welcher
- R¹: die oben angegebenen Bedeutungen hat,
gegebenenfalls in Gegenwart eines Säurebindemittels und gegebenenfalls in Gegenwart eines Verdünnungsmittels bei Temperaturen zwischen -20°C und +40°C umsetzt.

Es ist als äußerst überraschend zu bezeichnen, daß sich Trifluoracetessigsäureanilide der Formel (I) nach dem erfindungsgemäßen Verfahren in glatter Reaktion mit hohen Ausbeuten herstellen lassen. Eine solche Umsetzung war vor allem deshalb nicht zu erwarten, weil das als Ausgangssubstanz benötigte Trifluoracetessigsäure-chlorid auch bei niedrigen Temperaturen nur eine kurze Zeit stabil ist (vgl. Chem. Abstr. 1964, 2788 f und GB-A 931 689). Überraschend ist auch, daß solche Aniline der Formel (III), in denen R¹ für einen Rest der Formel -NH-SO₂-R³ steht, keine nennenswerte Reaktion des Trifluoracetessigsäure-chlorids mit der Sulfonylamino-Gruppe zeigen.

Das erfindungsgemäße Verfahren zeichnet sich durch eine Reihe von Vorteilen aus. So sind die benötigten Ausgangsstoffe in einfacher Weise und auch in größeren Mengen zugänglich. Ferner bereitet die Durchführung der Umsetzung und die Isolierung der gewünschten Substanzen keinerlei Probleme. Besonders günstig ist, daß die Trifluoracetessigsäure-anilide in hoher Ausbeute und großer Reinheit anfallen. Im übrigen ist das Verfahren breit anwendbar.

Verwendet man Trifluoracetessigsäurechlorid und 4-Cyano-2-fluor-5-methylsulfonylamino-anilin als Ausgangsstoffe, so kann der Verlauf des erfindungsgemäßen Verfahrens durch das folgende Formelschema veranschaulicht werden:

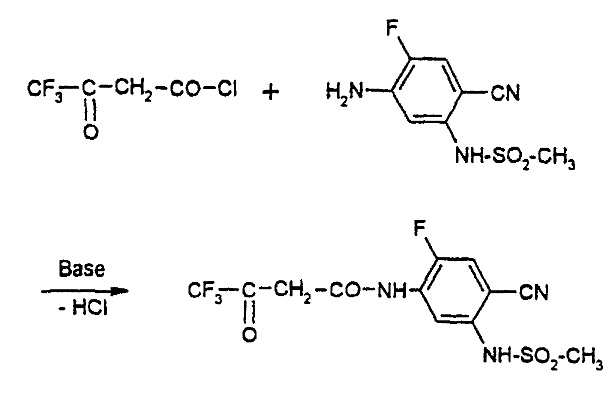

Das bei der Durchführung des erfindungsgemäßen Verfahrens als Ausgangssubstanz benötigte Trifluoracetessigsäurechlorid der Formel (II) ist bekannt (vgl. GB-A 931 689).

Die weiterhin bei der Durchführung des erfindungsgemäßen Verfahrens als Ausgangssubstanzen benötigten Aniline sind durch die Formel (III) allgemein definiert. Vorzugsweise verwendbar sind Verbindungen der Formel (III), in denen
- R¹: für Fluor, Chlor, Brom oder einen Rest der Formel 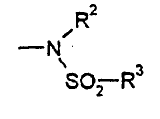 steht, worin
- R²: für Wasserstoff oder für gegebenenfalls durch Cyano, Halogen, Alkoxy mit 1 bis 4 Kohlenstoffatomen, Alkylthio mit 1 bis 4 Kohlenstoffatomen, Alkylcarbonyl mit 1 bis 4 Kohlenstoffatomen in der Alkylgruppe oder Alkylaminocarbonyl mit 1 bis 4 Kohlenstoffatomen im Alkylteil substituiertes Alkyl mit 1 bis 6 Kohlenstoffatomen steht und
- R³: für gegebenenfalls durch Cyano, Halogen, C₁-C₄-Alkoxy, C₁-C₄-Alkylthio, C₁-C₄-Alkyl-carbonyl, C₁-C₄-Alkoxy-carbonyl, C₁-C₄-Alkylamino-carbonyl oder Phenyl substituiertes Alkyl mit 1 bis 6 Kohlenstoffatomen steht,
oder für gegebenenfalls einfach bis dreifach, gleichartig oder verschieden durch Halogen, Cyano und/oder Alkyl mit 1 bis 4 Kohlenstoffatomen substituiertes Cycloalkyl mit 3 bis 6 Kohlenstoffatomen steht, oder für
gegebenenfalls einfach bis dreifach, gleichartig oder verschieden durch Halogen, Cyano, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenyl steht.

Besonders bevorzugt verwendbar sind Aniline der Formel (III), in denen
- R¹: für Fluor, Chlor oder einen Rest der Formel 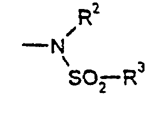 steht, worin
- R²: für Wasserstoff oder für jeweils gegebenenfalls durch Cyano, Fluor, Chlor, Methoxy, Ethoxy, Methylthio, Ethylthio, Acetyl, Propionyl, Methoxycarbonyl, Ethoxycarbonyl, Methylaminocarbonyl oder Ethylaminocarbonyl substituiertes Methyl, Ethyl, n- oder i-Propyl, n-, i-, soder t-Butyl steht und
- R³: für jeweils gegebenenfalls durch Cyano, Fluor, Chlor, Methoxy, Ethoxy, Methylthio, Ethylthio, Acetyl, Propionyl, Methoxycarbonyl, Ethoxycarbonyl, Methylaminocarbonyl, Ethylaminocarbonyl oder Phenyl substituiertes Methyl, Ethyl, n- oder i-Propyl, n-, i-, s- oder t-Butyl steht oder
für jeweils gegebenenfalls einfach bis dreifach, gleichartig oder verschieden durch Fluor, Chlor, Cyano, Methyl und/oder Ethyl substituiertes Cyclopropyl, Cyclopentyl oder Cyclohexyl steht, oder für
gegebenenfalls einfach bis dreifach, gleichartig oder verschieden durch Fluor, Chlor, Cyano, Methyl, Ethyl, Trifluormethyl, Methoxy, Ethoxy, Trifluormethoxy und/oder Difluormethoxy substituiertes Phenyl steht.

Die Aniline der Formel (III) sind bekannt oder lassen sich nach bekannten Methoden herstellen (vgl. EP-A 0 648 772).

Als Säureakzeptoren kommen bei der Durchführung des erfindungsgemäßen Verfahrens alle üblichen anorganischen oder organischen Basen in Betracht. Vorzugsweise verwendbar sind Alkalimetall- oder Erdalkalimetallcarbonate und -hydrogencarbonate, wie Natrium-, Kalium- oder Calciumcarbonat, Natrium-, Kalium- oder Calcium-hydrogencarbonat, sowie basische, organische Stickstoffverbindungen, wobei Trimethylamin, Triethylamin, Tripropylamin, Tributylamin, Ethyl-diisopropylamin, N,N-Dimethyl-cyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo-[4,3,0]-non-5-en (DBN), oder 1,8-Diazabicyclo[5,4,0]-undec-7-en (DBU) besonders bevorzugt sind.

Als Verdünnungsmittel kommen bei der Durchführung des erfindungsgemäßen Verfahrens alle üblichen inerten, organischen Solventien in Frage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; ferner Ether, wie Diethylether, Diisopropylether, Dioxan, Tetrahydrofuran, Ethylenglykoldimethyl- oder -diethylether; tert.-Butyl-methyl-ether oder tert.-Amyl-methylether, weiterhin Nitrile, wie Acetonitril, Propionitril oder Butyronitril, und auch Ester, wie Essigsäuremethylester oder Essigsäureethylester.

Die Reaktionstemperaturen können bei der Durchführung des erfindungsgemäßen Verfahrens in einem bestimmten Bereich variiert werden. Im allgemeinen arbeitet man bei Temperaturen zwischen -20°C und +40°C, vorzugsweise zwischen -10°C und +30°C.

Bei der Durchführung des erfindungsgemäßen Verfahrens arbeitet man im allgemeinen unter Atmosphärendruck. Es ist aber auch möglich, unter erhöhtem Druck zu arbeiten.

Bei der Durchführung des erfindungsgemäßen Verfahrens arbeitet man vorzugsweise unter Schutzgas, wie Stickstoff oder Argon.

Bei der Durchführung des erfindungsgemäßen Verfahrens setzt man auf 1 mol an Anilin der Formel (III) im allgemeinen 1 bis 3 mol, vorzugsweise 1 bis 1,9 mol, an Trifluoracetessigsäure-chlorid der Formel (II) sowie 1 bis 3 mol, vorzugsweise 1 bis 1,9 mol an Säurebindemittel ein. In einer bevorzugten Ausführungsform verfährt man in der Weise, daß man Anilin der Formel (III) und Säurebindemittel in einem Verdünnungsmittel vorlegt und dann Trifluoracetessigsaurechlorid der Formel (II) in einem Verdünnungsmittel hinzutropft. Die Aufarbeitung erfolgt nach üblichen Methoden. Im allgemeinen verfährt man in der Weise, daß man das kristallin anfallende Produkt abfiltriert, wäscht und trocknet. Eventuell noch vorhandene Verunreinigungen können nach üblichen Methoden entfernt werden (vgl. Herstellungsbeispiele).

Die erfindungsgemäß herstellbaren Trifluoracetessigsäure-anilide der Formel (I) können in der "Keto"-Form der Formel 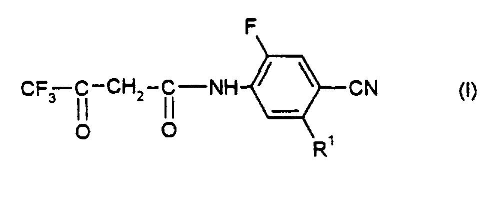 bzw. als Hydrat der Formel 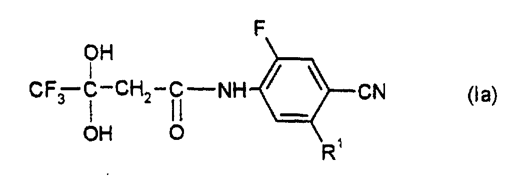 oder in der zu (I) tautomeren "Enol"-Form der Formel 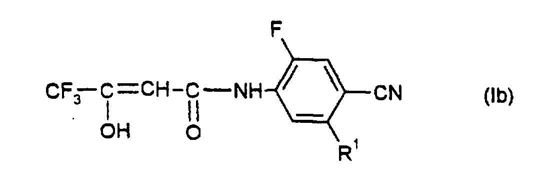 vorliegen. Der Einfachheit halber wird jeweils nur die "Keto"-Form angegeben.

Die erfindungsgemäß herstellbaren Trifluoracetessigsäure-anilide der Formel (I) sind wertvolle Zwischenprodukte zur Synthese von Uracil-Derivaten mit herbiziden Eigenschaften.

So lassen sich Uracil-Derivate der Formel 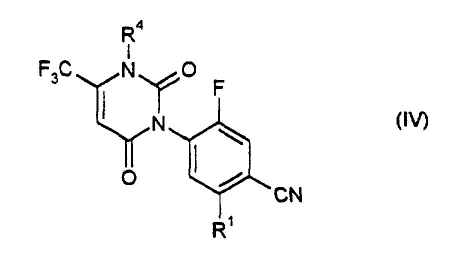 in welcher
- R¹: die oben angegebenen Bedeutungen hat und
- R⁴: für Wasserstoff, Hydroxy, Amino, für jeweils gegebenenfalls durch Hydroxy, Cyano, Fluor, Chlor, Methoxy oder Ethoxy substituiertes Methyl, Ethyl, n-oder i-Propyl, n-, i-, s- oder t-Butyl, Methoxy, Ethoxy, n- oder i-Propoxy, n-, i-, s- oder t-Butoxy, oder für jeweils gegebenenfalls durch Cyano, Fluor, Chlor oder Brom substituiertes Propenyl, Butenyl, Propinyl oder Butinyl steht,
herstellen, indem man Trifluoracetessigsäure-anilide der Formel 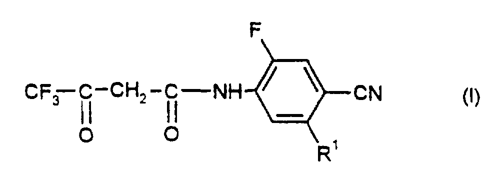 in welcher
- R¹: die oben angegebenen Bedeutungen hat,
mit Phosgen in Gegenwart eines Säurebindemittels, wie Pyridin oder 4-Dimethylaminopyridin, und in Gegenwart eines Verdünnungsmittels, wie Toluol oder Tetrahydrofuran, bei Temperaturen zwischen -20°C und +150°C umsetzt und die dabei entstehenden Phenyloxazin-dione der Formel 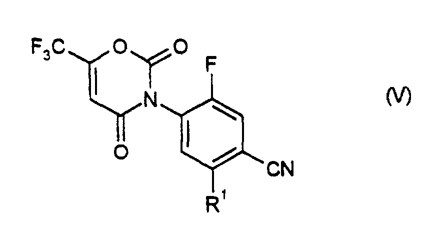 in welcher
- R¹: die oben angegebenen Bedeutungen hat,
mit Amino-Verbindungen der Formel

H₂N - R⁴ (VI)

in welcher
- R⁴: die oben angegebenen Bedeutungen hat,
gegebenenfalls in Gegenwart eines Säurebindemittels und gegebenenfalls in Gegenwart eines Verdünnungsmittels, wie Ethanol, bei Temperaturen zwischen -50°C und +80°C umsetzt.

Die Uracil-Derivate der Formel (IV) und deren Verwendung als Herbizide sind bekannt (vgl. EP-A 0 408 382, EP-A 0 648 749 und WO 95-32 952).

Das erfindungsgemäße Verfahren wird durch die folgenden Beispiele veranschaulicht.

### Herstellungsbeispiele

### Beispiel 1

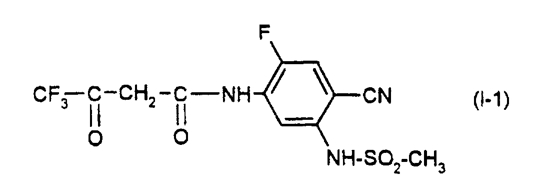

In eine Suspension von 10,3 g (45 mmol) 4-Cyano-2-fluor-5-methylsulfonylaminoanilin und 4,28 g (54 mmol) Pyridin in 110 ml Methylenchlorid werden unter Argonatmosphäre bei 20 bis 22°C unter Rühren innerhalb von 15 Minuten 40,4 g einer 35 %-igen Lösung von Trifluoracetessigsäure-chlorid (81 mmol) in Methylenchlorid eingetropft. Nach beendeter Zugabe wird der Tropftrichter mit 10 ml Methylenchlorid nachgespült, die ebenfalls in das Reaktionsgemisch getropft werden. Das Fortschreiten der Umsetzung wird durch Dünnschichtchromatographie kontrolliert. Nach 15-minütiger Reaktionszeit wird aufgearbeitet, indem man auf 0°C kühlt, den angefallenen Niederschlag abfiltriert, mit Methylenchlorid nachwäscht und trocknet. Man erhält 15 g einer pulverförmigen Substanz, die mit Wasser versetzt wird. Das entstehende Gemisch wird dreimal mit Essigsäureethylester extrahiert. Die vereinigten organischen Phasen werden nach dem Trocknen über Natriumsulfat unter vermindertem Druck eingeengt. Das verbleibende Produkt wird unter Vakuum getrocknet. Man erhält auf diese Weise 12,2 g (73,8 % der Theorie) an N-(4-Cyano-2-fluor-5-methylsulfonylamino-phenyl)-4,4,4-trifluor-3-oxo-butyramid in Form einer Festsubstanz vom Schmelzpunkt 186 bis 191°C.

### Beispiel 2

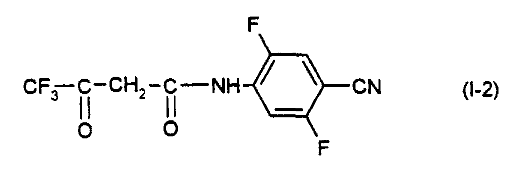

In ein auf 10°C gekühltes Gemisch aus 169,5 g (1,1 mol) 2,5-Difluor-4-cyano-anilin, 104,5 g Pyridin (1,32 mol) und 640 ml Methylenchlorid werden unter Argonatmosphäre und unter Rühren innerhalb von 25 Minuten 713 g einer Lösung von Trifluoracetessigsäure-chlorid (1,43 mol, 35 %ige Lösung) in Methylenchlorid eingetropft. Nach beendeter Zugabe wird der Tropftrichter mit 20 ml Methylenchlorid nachgespült, die ebenfalls in das Reaktionsgemisch getropft werden. Das Fortschreiten der Reaktion wird durch Dünnschichtchromatographie kontrolliert. Nach einstündigem Nachrühren bei 22°C wird das Reaktionsgemisch auf 0°C gekühlt und 15 Minuten bei dieser Temperatur gerührt. Der ausgefallene Niederschlag wird abfiltriert, mit Methylenchlorid gewaschen und getrocknet. Man erhält 209,3 g eines Rohproduktes, das mit Wasser versetzt wird. Das entstehende Gemisch wird mehrfach mit Essigsäureethylester extrahiert. Die wäßrige Phase wird mit konzentrierter Salzsäure stark angesäuert und erneut mit Essigsäureethylester extrahiert. Die vereinigten organischen Phasen werden mit Wasser gewaschen und dann über Natriumsulfat getrocknet. Die nach der Filtration verbleibende Lösung wird unter vermindertem Druck eingeengt. Das verbleibende Produkt wird unter Vakuum getrocknet. Man erhält auf diese Weise 150,4 g (47,1 % der Theorie) an N-(4-Cyano-2,5-difluorphenyl)-4,4,4-trifluor-3-oxo-butyramid in Form einer weißen Festsubstanz vom Schmelzpunkt 176 bis 181°C.

Aus der Mutterlauge wird durch erneute Aufarbeitung in der angegebenen Weise weiteres Produkt in Form des Hydrates der Verbindung der Formel (1-2) isoliert. Die Gesamtausbeute beträgt 54 % der Theorie.

### Beispiel 3

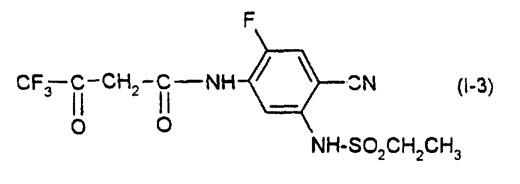

Analog Beispiel 1 wird aus 4-Cyano-2-fluor-5-ethylsulfonylamino-anilin N-(4-Cyano-2-fluor-5-ethylsulfonylamino-phenyl)-4,4,4-trifluor-3-oxobutyramid vom Schmelzpunkt 185 bis 187°C hergestellt.

### Verwendungsbeispiel A

Herstellung der Verbindung der Formel

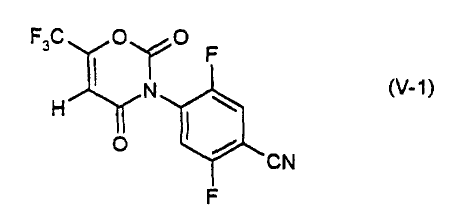

120 g einer 20%igen Lösung von Phosgen in Toluol werden bei 40°C unter Rühren zu einer Mischung aus 60 g (0,20 Mol) N-(4-Cyano-2,5-difluor-phenyl)-3-oxo-4,4,4-trifluor-1-butansäureamid, 40 ml Pyridin, 4 g 4-Dimethylamino-pyridin und 1,5 Litern Toluol tropfenweise gegeben. Die Reaktionsmischung wird dann noch 4 Stunden bei 40°C gerührt. Anschließend wird überschüssiges Phosgen mit Stickstoff ausgeblasen. Das verbleibende Gemisch wird dreimal mit Wasser gewaschen, über Natriumsulfat getrocknet und filtriert. Vom Filtrat wird das Lösungsmittel im Wasserstrahlvakuum sorgfältig abdestilliert.

Man erhält 63,7 g (77,5% der Theorie) 3-(4-Cyano-2,5-difluor-phenyl)-3,4-dihydro-6-trifluormethyl-2H-1,3-oxazin-2,4-dion als zähe Masse, welche allmählich durchkristallisiert.

Herstellung der Verbindung der Formel

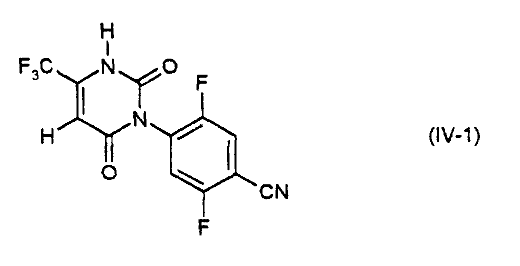

7 ml einer 25%igen wässrigen Lösung von Ammoniak (0,10 mol NH₃) werden bei Raumtemperatur (ca. 20°C) tropfenweise unter Rühren zu einer Mischung aus 15,9 g (0,05 mol) 3-(4-Cyano-2,5-difluor-phenyl)-3,4-dihydro-6-trifluormethyl-2H-1,3-oxazin-2,4-dion und 100 ml Ethanol gegeben. Die Reaktionsmischung wird 20 Stunden bei Raumtemperatur gerührt. Dann wird im Wasserstrahlvakuum eingeengt und der Rückstand in Essigsäureethylester aufgenommen. Die entstehende Lösung wird, mit Wasser gewaschen, über Natriumsulfat getrocknet und filtriert. Das Filtrat wird unter vermindertem Druck eingeengt, der Rückstand mit wenig Isopropanol digeriert und das kristallin anfallende Produkt durch Absaugen isoliert.

Man erhält 11,8 g (74 % der Theorie) 1-(4-Cyano-2,5-difluor-phenyl)-3,6-dihydro-2,6-dioxo-4-trifluormethyl-1(2H)-pyrimidin vom Schmelzpunkt 234°C.

## Patentansprüche

1. Verfahren zur Herstellung von Trifluoracetessigsäure-aniliden der Formel 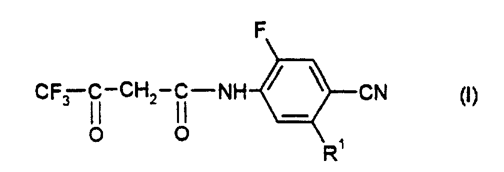 in welcher
R¹ für Halogen oder einen Rest der Formel 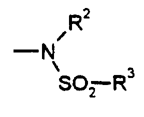 steht, worin
R² für Wasserstoff oder gegebenenfalls substituiertes Alkyl steht und
R³ für gegebenenfalls substituiertes Alkyl, gegebenenfalls substituiertes Cycloalkyl oder für gegebenenfalls substituiertes Aryl steht,
**dadurch gekennzeichnet, daß** man Trifluoracetessigsäure-chlorid der Formel 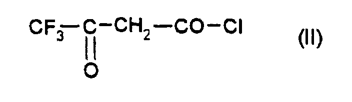 mit Anilinen der Formel 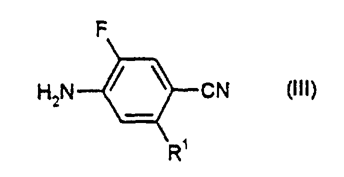 in welcher
R¹ die oben angegebenen Bedeutungen hat,
gegebenenfalls in Gegenwart eines Säurebindemittels und gegebenenfalls in Gegenwart eines Verdünnungsmittels bei Temperaturen zwischen -20°C und +40°C umsetzt.

2. Verfahren gemäß Anspruch 1, **dadurch gekennzeichnet, daß** man als Ausgangsstoffe Aniline der Formel (III) einsetzt, in denen
R¹ für Fluor, Chlor, Brom oder einen Rest der Formel 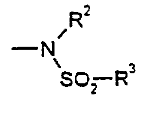 steht, worin
R² für Wasserstoff oder für gegebenenfalls durch Cyano, Halogen, Alkoxy mit 1 bis 4 Kohlenstoffatomen, Alkylthio mit 1 bis 4 Kohlenstoffatomen, Alkylcarbonyl mit 1 bis 4 Kohlenstoffatomen in der Alkylgruppe oder Alkylaminocarbonyl mit 1 bis 4 Kohlenstoffatomen im Alkylteil substituiertes Alkyl mit 1 bis 6 Kohlenstoffatomen steht und
R³ für gegebenenfalls durch Cyano, Halogen, C₁-C₄-Alkoxy, C₁-C₄-Alkylthio, C₁-C₄-Alkyl-carbonyl, C₁-C₄-Alkoxy-carbonyl, C₁-C₄-Alkylamino-carbonyl oder Phenyl substituiertes Alkyl mit 1 bis 6 Kohlenstoffatomen steht,
oder für gegebenenfalls einfach bis dreifach, gleichartig oder verschieden durch Halogen, Cyano und/oder Alkyl mit 1 bis 4 Kohlenstoffatomen substituiertes Cycloalkyl mit 3 bis 6 Kohlenstoffatomen steht, oder für gegebenenfalls einfach bis dreifach, gleichartig oder verschieden durch Halogen, Cyano, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenyl steht.

3. Verfahren gemäß Anspruch 1, **dadurch gekennzeichnet, daß** man als Ausgangsstoffe Aniline der Formel (III), in denen
R¹ für Fluor, Chlor oder einen Rest der Formel 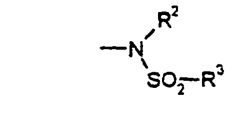 steht, worin
R² für Wasserstoff oder für jeweils gegebenenfalls durch Cyano, Fluor, Chlor, Methoxy, Ethoxy, Methylthio, Ethylthio, Acetyl, Propionyl, Methoxycarbonyl, Ethoxycarbonyl, Methylaminocarbonyl oder Ethylaminocarbonyl substituiertes Methyl, Ethyl, n- oder i-Propyl, n-, i-, s- oder t-Butyl steht und
R³ für jeweils gegebenenfalls durch Cyano, Fluor, Chlor, Methoxy, Ethoxy, Methylthio, Ethylthio, Acetyl, Propionyl, Methoxycarbonyl, Ethoxycarbonyl, Methylaminocarbonyl, Ethylaminocarbonyl oder Phenyl substituiertes Methyl, Ethyl, n- oder i-Propyl, n-, i-, s- oder t-Butyl steht oder für jeweils gegebenenfalls einfach bis dreifach, gleichartig oder verschieden durch Fluor, Chlor, Cyano, Methyl und/oder Ethyl substituiertes Cyclopropyl, Cyclopentyl oder Cyclohexyl steht, oder für
gegebenenfalls einfach bis dreifach, gleichartig oder verschieden durch Fluor, Chlor, Cyano, Methyl, Ethyl, Trifluormethyl, Methoxy, Ethoxy, Trifluormethoxy und/oder Difluormethoxy substituiertes Phenyl steht.

4. Verfahren gemäß Anspruch 1, **dadurch gekennzeichnet, daß** man als Ausgangsstoff das Anilin der Formel 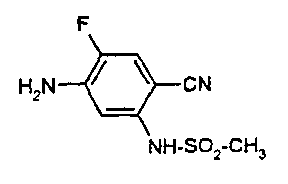 einsetzt.

5. Verfahren gemäß Anspruch 1, **dadurch gekennzeichnet, daß** man als Ausgangsstoff das Anilin der Formel 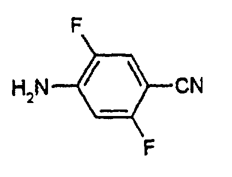 einsetzt.

6. Verfahren gemäß Anspruch 1, **dadurch gekennzeichnet, daß** man als Ausgangsstoff das Anilin der Formel 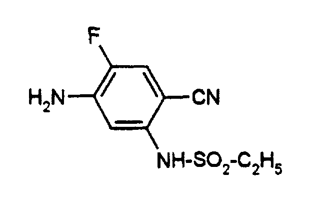 einsetzt.

7. Verfahren zur Herstellung von Uracil-Derivaten der Formel 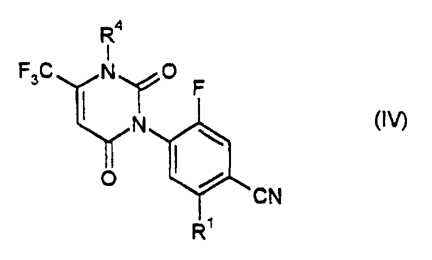 in welcher
R¹ die im Anspruch 1 angegebenen Bedeutungen hat und
R⁴ für Wasserstoff, Hydroxy, Amino, für jeweils gegebenenfalls durch Hydroxy, Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, oder für jeweils gegebenenfalls durch Cyano, Fluor, Chlor oder Brom substituiertes Propenyl, Butenyl, Propinyl oder Butinyl steht,
**dadurch gekennzeichnet, daß** man Trifluoracetessigsäure-anilide der Formel 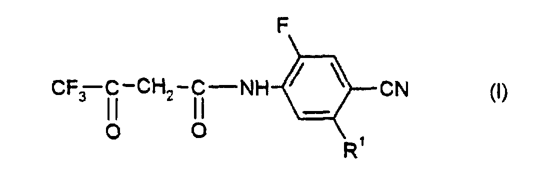 in welcher
R¹ die oben angegebenen Bedeutungen hat,
mit Phosgen in Gegenwart eines Säurebindemittels und in Gegenwart eines Verdünnungsmittels bei Temperaturen zwischen -20°C und +150°C umsetzt und die dabei entstehenden Phenyloxazin-dione der Formel 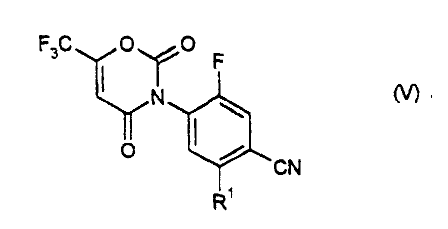 in welcher
R¹ die oben angegebenen Bedeutungen hat,
mit Amino-Verbindungen der Formel
H₂N - R⁴ (VI)
in welcher
R⁴ die oben angegebenen Bedeutungen hat,
gegebenenfalls in Gegenwart eines Säurebindemittels und gegebenenfalls in Gegenwart eines Verdünnungsmittels bei Temperaturen zwischen -50°C und +80°C umsetzt.

## Claims

1. Process for preparing trifluoroacetoacet-anilides of the formula 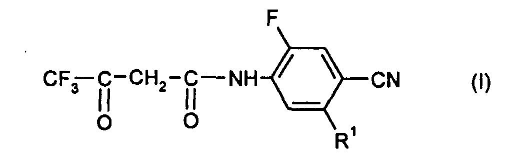 in which
R¹ represents halogen or a radical of the formula 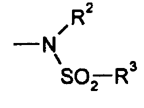 in which
R² represents hydrogen or optionally substituted alkyl and
R³ represents optionally substituted alkyl, optionally substituted cycloalkyl or represents optionally substituted aryl,
**characterized in that** trifluoroacetoacetyl chloride of the formula 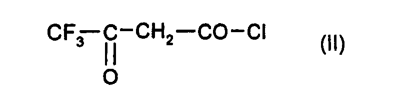 is reacted with anilines of the formula 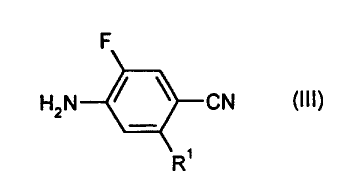 in which
R¹ is as defined above,
if appropriate in the presence of an acid binder and if appropriate in the presence of a diluent, at temperatures between -20°C and +40°C.

2. Process according to Claim 1, **characterized in that** the starting materials used are anilines of the formula (III) in which
R¹ represents fluorine, chlorine, bromine or a radical of the formula 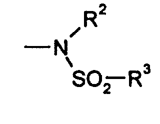 in which
R² represents hydrogen or represents alkyl having 1 to 6 carbon atoms which is optionally substituted by cyano, halogen, alkoxy having 1 to 4 carbon atoms, alkylthio having 1 to 4 carbon atoms, alkylcarbonyl having 1 to 4 carbon atoms in the alkyl group or alkylaminocarbonyl having 1 to 4 carbon atoms in the alkyl moiety and
R³ represents alkyl having 1 to 6 carbon atoms, which is optionally substituted by cyano, halogen, C₁-C₄-alkoxy, C₁-C₄-alkylthio, C₁-C₄-alkylcarbonyl, C₁-C₄-alkoxycarbonyl, C₁-C₄-alkylaminocarbonyl or phenyl,
or represents cycloalkyl having 3 to 6 carbon atoms which is optionally mono- to trisubstituted by identical or different substituents selected from the group consisting of halogen, cyano and alkyl having 1 to 4 carbon atoms, or represents
phenyl which is optionally mono- to trisubstituted by identical or different substituents selected from the group consisting of halogen, cyano, alkyl having 1 to 4 carbon atoms, halogenoalkyl having 1 or 2 carbon atoms and 1 to 5 halogen atoms, alkoxy having 1 to 4 carbon atoms and/or halogenoalkoxy having 1 or 2 carbon atoms and 1 to 5 halogen atoms.

3. Process according to Claim 1, **characterized in that** the starting materials used are anilines of the formula (III) in which
R¹ represents fluorine, chlorine or a radical of the formula 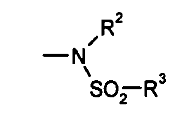 in which
R² represents hydrogen or represents methyl, ethyl, n- or i-propyl, n-, i-, s- or t-butyl, each of which is optionally substituted by cyano, fluorine, chlorine, methoxy, ethoxy, methylthio, ethylthio, acetyl, propionyl, methoxycarbonyl, ethoxycarbonyl, methylaminocarbonyl or ethylaminocarbonyl and
R³ represents methyl, ethyl, n- or i-propyl, n-, i-, s- or t-butyl, each of which is optionally substituted by cyano, fluorine, chlorine, methoxy, ethoxy, methylthio, ethylthio, acetyl, propionyl, methoxycarbonyl, ethoxycarbonyl, methylaminocarbonyl, ethylaminocarbonyl or phenyl or
represents cyclopentyl, cyclohexyl or cyclopropyl, each of which is optionally mono- to trisubstituted by identical or different substituents selected from the group consisting of fluorine, chlorine, cyano, methyl and ethyl, or represents
phenyl which is optionally mono- to trisubstituted by identical or different substituents selected from the group consisting of fluorine, chlorine, cyano, methyl, ethyl, trifluoromethyl, methoxy, ethoxy, trifluoromethoxy and difluoromethoxy.

4. Process according to Claim 1, **characterized in that** the starting material used is the aniline of the formula

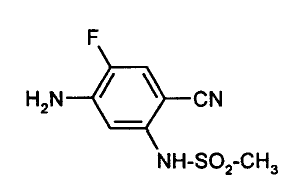

5. Process according to Claim 1, **characterized in that** the starting material used is the aniline of the formula

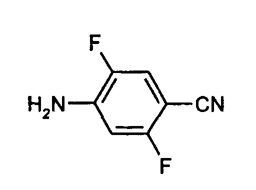

6. Process according to Claim 1, **characterized in that** the starting material used is the aniline of the formula

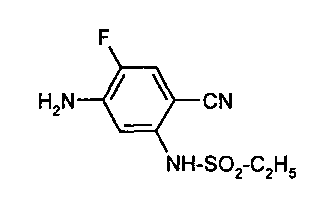

7. Process for preparing uracil derivatives of the formula 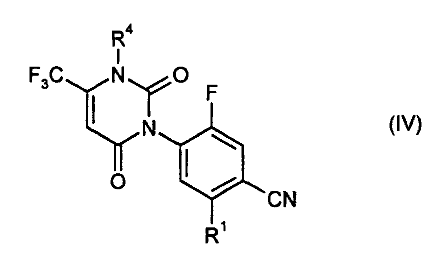 in which
R¹ is as defined in Claim 1 and
R⁴ represents hydrogen, hydroxyl, amino, represents methyl, ethyl, n- or i-propyl, n-, i-, s- or t-butyl, methoxy, ethoxy, n- or i-propoxy, n, i-, s- or t-butoxy, each of which is optionally substituted by hydroxyl, cyano, fluorine, chlorine, methoxy or ethoxy, or represents propenyl, butenyl, propinyl or butinyl, each of which is optionally substituted by cyano, fluorine, chlorine or bromine,
**characterized in that** trifluoroacetoacet-anilides of the formula 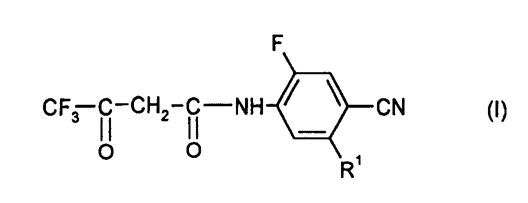 in which
R¹ is as defined above,
are reacted with phosgene in the presence of an acid binder and in the presence of a diluent at temperatures between -20°C and +150°C, and the resulting phenyloxazine-diones of the formula 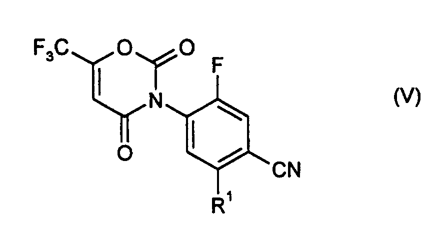 in which
R¹ is as defined above
are reacted with amino compounds of the formula
H₂N - R⁴ (VI)
in which
R⁴ is as defined above,
if appropriate in the presence of an acid binder and if appropriate in the presence of a diluent, at temperatures between -50°C and +80°C.

## Revendications

1. Procédé de production d'anilides d'acide trifluoracétylacétique de formule 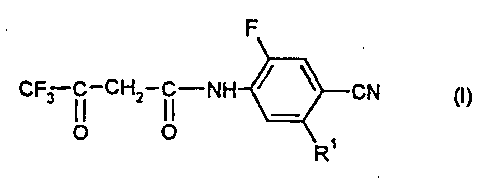 dans laquelle
R¹ représente un halogène ou un reste de formule 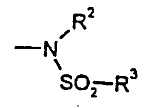 dans laquelle
R² représente l'hydrogène ou un groupe alkyle éventuellement substitué
et
R³ représente un groupe alkyle éventuellement substitué, un groupe cycloalkyle éventuellement substitué ou un groupe aryle éventuellement substitué,
**caractérisé en ce qu'**on fait réagir le chlorure d'acide trifluoracétylacétique de formule 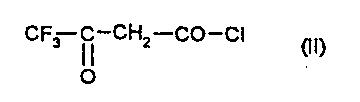 avec des anilines de formule 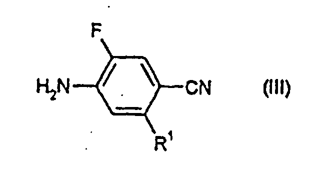 dans laquelle
R¹ a les définitions indiquées ci-dessus,
éventuellement en présence d'un accepteur d'acide et, le cas échéant, en présence d'un diluant, à des températures comprises entre -20°C et +40°C.

2. Procédé suivant la revendication 1, **caractérisé en ce qu'**on utilise comme composés de départ des anilines de formule (III), dans lesquelles
R¹ représente le fluor, le chlore, le brome ou un reste de formule 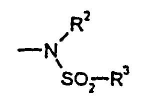 dans laquelle
R² représente l'hydrogène ou un groupe alkyle ayant 1 à 6 atomes de carbone, éventuellement substitué par un radical cyano, halogéno, alkoxy ayant 1 à 4 atomes de carbone, alkylthio ayant 1 à 4 atomes de carbone, alkylcarbonyle ayant 1 à 4 atomes de carbone dans le groupe alkyle ou alkylaminocarbonyle ayant 1 à 4 atomes de carbone dans la partie alkyle, et
R³ représente un groupe alkyle ayant 1 à 6 atomes de carbone facultativement substitué par un radical cyano, halogéno, alkoxy en C₁ à C₄, alkylthio en C₁ à C₄, (alkyle en C₁ à C₄)carbonyle, (alkoxy en C₁ à C₄)carbonyle, (alkylamino en C₁ à C₄)-carbonyle ou phényle,
ou bien un groupe cycloalkyle de 3 à 6 atomes de carbone éventuellement substitué une à trois fois identiques ou différentes par un radical halogéno, cyano et/ou alkyle ayant 1 à 4 atomes de carbone, ou bien
un groupe phényle éventuellement substitué une à trois fois identiques ou différentes par un radical halogéno, cyano, alkyle ayant 1 à 4 atomes de carbone, halogénalkyle ayant 1 ou 2 atomes de carbone et 1 à 5 atomes d'halogènes, alkoxy ayant 1 à 4 atomes de carbone et/ou halogénalkoxy ayant 1 ou 2 atomes de carbone et 1 à 5 atomes d'halogènes.

3. Procédé suivant la revendication 1, **caractérisé en ce qu'**on utilise comme composés de départ des anilines de formule (III), dans lesquelles
R¹ représente le fluor, le chlore ou un reste de formule 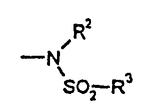 dans laquelle
R² représente l'hydrogène ou un groupe méthyle, éthyle, n-propyle, isopropyle, n-butyle, isobutyle, sec.-butyle ou tertio-butyle, chacun étant éventuellement substitué par un radical cyano, fluoro, chloro, méthoxy, éthoxy, méthylthio, éthylthio, acétyle, propionyle, méthoxycarbonyle, éthoxycarbonyle, méthylaminocarbonyle ou éthylaminocarbonyle et
R³ représente un groupe méthyle, éthyle, n-propyle, isopropyle, n-butyle, isobutyle, sec.-butyle ou tertio-butyle, chacun étant éventuellement substitué par un radical cyano, fluoro, chloro, méthoxy, éthoxy, méthylthio, éthylthio, acétyle, propionyle, méthoxycarbonyle, éthoxycarbonyle, méthylaminocarbonyle, éthylaminocarbonyle ou phényle, ou
un groupe cyclopropyle, cyclopentyle ou cyclohexyle, chacun étant éventuellement substitué une à trois fois identiques ou différentes par un radical fluoro, chloro, cyano, méthyle et/ou éthyle, ou bien
un groupe phényle éventuellement substitué une à trois fois identiques ou différentes par un radical fluoro, chloro, cyano, méthyle, éthyle, trifluorométhyle, méthoxy, éthoxy, trifluorométhoxy et/ou difluorométhoxy.

4. Procédé suivant la revendication 1, **caractérisé en ce qu'**on utilise comme composé de départ l'aniline de formule

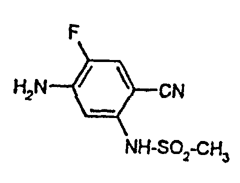

5. Procédé suivant la revendication 1, **caractérisé en ce qu'**on utilise comme composé de départ l'aniline de formule

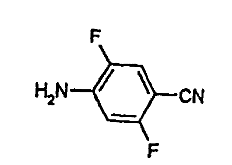

6. Procédé suivant la revendication 1, **caractérisé en ce qu'**on utilise comme composé de départ l'aniline de formule

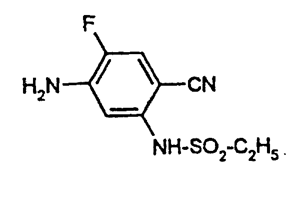

7. Procédé de production de dérivés d'uracile de formule 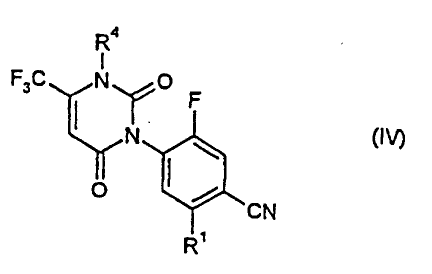 dans laquelle
R¹ a les définitions indiquées dans la revendication 1 et
R⁴ représente l'hydrogène, un groupe hydroxy, amino, un groupe méthyle, éthyle, n-propyle, isopropyle, nbutyle, isobutyle, sec.-butyle, tertio-butyle, méthoxy, éthoxy, n-propoxy, isopropoxy, n-butoxy, isobutoxy, sec.-butoxy ou tertio-butoxy, chacun étant éventuellement substitué par un radical hydroxy, cyano, fluoro, chloro, méthoxy ou éthoxy, ou bien un groupe propényle, butényle, propynyle ou butynyle, chacun éventuellement substitué par un radical cyano, fluoro, chloro ou bromo,
**caractérisé en ce qu'**on fait réagir des anilides d'acide trifluoracétylacétique de formule 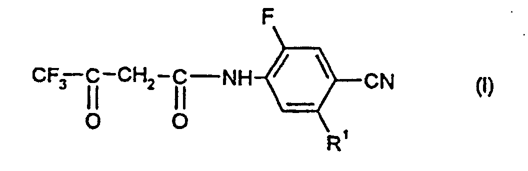 dans laquelle
R¹ a les définitions indiquées ci-dessus,
avec le phosgène en présence d'un accepteur d'acide et en présence d'un diluant à des températures comprises entre -20°C et +150°C et on fait réagir les phényloxazine-diones ainsi produites de formule 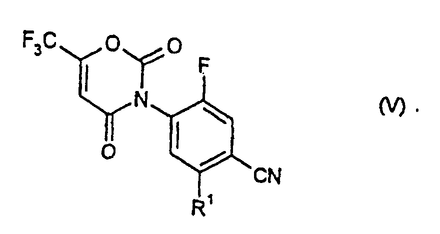
dans laquelle
R¹ a les définitions indiquées ci-dessus,
avec des composés aminés de formule
H₂N-R⁴ (VI)
dans laquelle
R⁴ a les définitions indiquées ci-dessus,
le cas échéant en présence d'un accepteur d'acide et en présence éventuelle d'un diluant, à des températures comprises entre -50°C et +80°C.
